# EUROPÄISCHE PATENTSCHRIFT

(11) **EP 0 557 701 B1**
(45) Veröffentlichungstag und Bekanntmachung des Hinweises auf die Patenterteilung: **21.04.1999**
(21) Anmeldenummer: 93100613.4
(22) Anmeldetag: 16.01.1993
(51) Int. Cl.: A61L 2/26

(54) **Gerät zum Testen von Dampfsterilisatoren**
Test device for steam sterilizers
Dispositif de test pour la stérilisation à la vapeur

(30) Priorität: 24.02.1992 DE 9202347 U
(43) Veröffentlichungstag der Anmeldung: 01.09.1993
(73) Patentinhaber: Wolf, Hans, D-74821 Mosbach (DE)
(72) Erfinder: Wolf, Hans, D-74821 Mosbach (DE)
(74) Vertreter: Wolf, Günter, Dipl.-Ing.

(56) Entgegenhaltungen:
- WO-A-87/01039
- WO-A-88/01372
- DE-U- 9 202 347

## Beschreibung

Die Erfindung betrifft ein Gerät zum Testen von Dampfsterilisatoren mit Hilfe eines Testmaterialstapels, gemäß Oberbegriff des Patentanspruches 1. Solche Geräte bestehen im Prinzip aus zwei mit Durchbrechungen versehenen, den Stapelaufnahmeraum begrenzenden Stapelhalteplatten, die relativ zueinander und in bezug auf den Testmaterialstapel in Parallel- und Spannstellung bring- und fixierbar sind. Geräte dieser Art sind bspw. nach der GB-A-2143322 und nach dem DE-U- 85 23 448 bzw. nach der EP-A-233 226 bekannt. Um bei der Dampfsterilisation von Wäschestapeln die einwandfreie Funktion der Dampfsterilisatoren zu gewährleisten, müssen diese permanent mittels Testmaterialstapeln getestet werden, wobei diese Stapel in Geräte der gattungsgemäßen Art eingelegt und zusammen mit diesen in den zu testenden Dampfsterilisator eingebracht werden. Nach vollzogener Teststerilisation wird das Gerät entnommen, muß im geöffneten Zustand auskühlen, und dann wird der eigentliche Testmaterialbogen hinsichtlich seines Sterilitätsgrades untersucht. In seiner tatsächlichen Ausführungsform besteht das Gerät nach dem DE-U-85 23 448 aus einem die eine Halteplatte bildenden Bodenteil mit an seinen Ecken angeordneten Standfüßen, die nach oben gerichtet, Führungszapfen für das die andere Halteplatte bildende, mit einem Handgriff versehene Deckelteil bilden. Um das Boden- und Deckelteil mit dem darin befindlichen Testmaterialstapel bis zu einem gewissen Grade unter mechanische Spannung setzen zu können, sind an zwei Seiten der rahmenartig ausgebildeten Teile federbelastete Spannverschlüsse angeordnet. Die Be- und Entladung eines solchen Gerätes ist aufgrund dieser Konstruktion ziemlich umständlich und mühsam, da diese nur bei völlig abgenommenem Deckelteil erfolgen können, wobei nach einer durchgeführten Teststerilisation die relativ hohe Temperatur des ganzen Gerätes zu berücksichtigen ist. Da das Gerät mit dem Teststapel in horizontaler Stellung benutzt werden muß, dauert die Abkühlung insbesondere des Teststapels relativ lange (ca. 1/2 Stunde). Insbesondere führen die federbelasteten Spannverschlüsse sowohl beim Schließen als auch beim Öffnen zu einer ziemlichen "Fummelei", zumal beim Öffnen die Spannvezschlüsse natürlich auch entsprechend heiß sind.

Der Erfindung liegt die Aufgabe zugrunde, das Gerät der gattungsgemäßen Art dahingehend zu verbessern, daß bei vergleichbar einfachem Aufbau nicht nur das Gerät selbst, sondern auch der Teststapel wesentlich günstiger und einfacher zu handhaben ist, und dies verbunden mit der Maßgabe, den Testmaterialstapel im Dampfsterilisator auch vertikal anordnen zu können, wobei außerdem das Gerät ohne Entfernung eines Geräteteiles zu öffnen und das Gerät zusammen mit dem Teststapel einer schnelleren Abkühlung zugänglich sein soll.

Diese Aufgabe ist mit einem Gerät der gattungsgemäßen Art nach der Erfindung durch die im Kennzeichen des Patentanspruches 1 angeführten Merkmale gelöst. Vorteilhafte Ausgestaltungen ergeben sich nach den Unteransprüchen.

Durch diese erfindungsgemäße Ausbildung kann der Teststapel problemlos bei geöffnetem Gerät von oben und leicht schräg zwischen die aufgespreizten Stapelhalteplatten eingebracht werden, die danach einfach zusammengedrückt und in Schließstellung durch die selbsttätig einrastenden Verrastungselementenpaare zusammengehalten werden. Der erforderliche Preßdruck auf den Teststapel ergibt sich dabei automatisch durch das noch näher zu erläuternde elastisch-flächenwirksame Druckelement im Stapelaufnahmeraum. Zum Öffnen des Gerätes nach erfolgter Teststerilisation sind lediglich die Verrastungselementenpaare zu lösen, wodurch die schwenkbar gelagerte Stapelhalteplatte selbsttätig aufspringt und auch der Teststapel drucklos wird. Zum Auskühlen braucht der Teststapel nicht entnommen zu werden, da sich dieser, ähnlich wie Buchseiten, bis zu einem gewissen Grade aufspreizt, die Wärme nach oben schneller entweichen und der Stapel ausreichend austrocknen kann. Um Schwenkbegrenzungsanschläge und gelenkige Lagerungen für beide Stapelhalteplatten zu vermeiden, wird eine Ausführungsform bevorzugt, bei der die eine Stapelhalteplatte fest und die andere schwenkbar mit dem Gerätefuß verbunden ist.

Vorteilhafte weitere Ausgestaltungen bestehen in folgendem: Durch eine Ausbildung nach Anspruch 2 wird es entbehrlich, den Gerätefuß aus gelochtem Blech herzustellen, was notwendig wäre, um den Teststapel auch von unten für den Dampf zugänglich zu machen. Außerdem ist der Materialaufwand für derartige Winkelstücke kleiner.

Eine Ausbildung gemäß Anspruch 3 hat den Vorteil, daß das ganze Gerät sehr einfach beidhändig mit den Fingerspitzen unterfaßt und getragen werden kann, wobei im heißen Zustand nur sehr schmale Griffstege von Blechstärkendicke unterfaßt werden müssen. Außerdem kann durch diese Ausbildung das Gerät auch horizontal aufgestellt werden, da hierfür die vorerwähnten Winkelstücke und die Plattengriffe als Gerätefüße dienen.

Bei der Ausbildung der Vorrichtung bzw. der Verrastungselementenpaare gemäß Anspruch 4 besteht die Schlitzführung dabei aus einem sehr kurzen nach oben orientierten Teil, mit dem die Verrastung in Schließstellung erfolgt, und aus einem etwa horizontal orientierten Teil, der den Öffnungsweg für die eine Halteplatte vorgibt. Beim Schließen fällt die Lasche mit ihrem kurzen Führungsteil selbsttätig auf den Stellzapfen, und zum Öffnen sind die Laschen nur kurz von unten anzutippen, wodurch die schwenkbare Halteplatte bis zum Anschlag der Schlitzführung ebenfalls automatisch aufschwenkt.

Das Druckelement kann in unterschiedlicher Ausführung, d.h., im Sinne der Unteransprüche 5, 6 ausgebildet werden. Eine Druckelementenausbildung im Sinne des Anspruches 6 ist insofern besonders vorteilhaft, weil solche Platten eine besondere Druckplatte entbehrlich machen, an dieser per se durch die Zungenausprägungen Durchbrechungen vorhanden sind und außerdem solche Elemente bzw. Bleche aus anderen Bereichen handelsverfügbar sind.

Eine ebenfalls im Rahmen der Handhabungserleichterung liegende Ausgestaltung ergibt sich nach Unteranspruch 7. Hierdurch ergibt sich eine versetzte Zuordnung der beiden Stapelhälften, d.h., das mittig darin befindliche und eigentlich interessierende Testblatt wird mit seinem oberen Rand einem sofortigen Zugriff zugänglich und kann direkt herausgezogen werden.

Um eine Seitenbegrenzung für den Stapelaufnahmeraum und günstige Anbringungsmöglichkeiten für die Verrastungselementenpaare zu schaffen, sind die Seitenränder beider Stapelhalteplatten zum Stapelaufnahmeraum hin rechtwinklig an den Stapelplattenebenen abgekröpft.

Das erfindungsgemäße Gerät wird nachfolgend anhand der zeichnerischen Darstellung von Ausführungsbeispielen näher erläutert.

Es zeigt
- Fig. 1: das Gerät in Seitenansicht und in Schließstellung;
- Fig. 2: das Gerät gemäß Fig. 1 in Öffnungsstellung;
- Fig. 3: das Gerät in Vorderansicht;
- Fig. 4,5: verkleinert in Vorder- und Seitenansicht eine Ausführungsform der Druckplatte;
- Fig. 6: im Teilschnitt eine andere Ausführungsform der Druck- und Stapelhalteplatte und
- Fig. 7: eine Darstellung gemäß Fig. 2 in einer besonderen Ausführungsform und mit dem eingesetzten Teststapel.

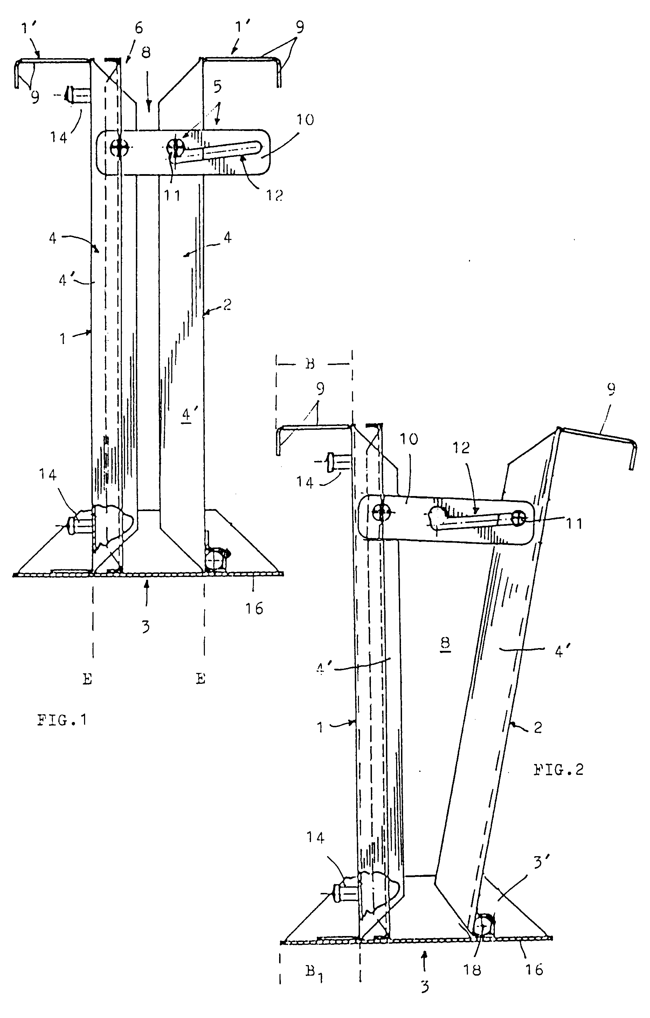

Wie aus den Fig. 1 bis 3 ersichtlich, weist das Gerät zwei mit Durchbrechungen 7 versehenen, den Stapelaufnahmeraum 8 begrenzenden Stapelhalteplatten 1, 2 auf, die relativ zueinander und in bezug auf den Testmaterialstapel in Parallel- und Spannstellung bring- und fixierbar sind.

Für dieses Gerät ist nun wesentlich, daß die beiden Stapelhalteplatten 1, 2 buchdeckelartig einander zugeordnet und buchdeckelrückenseitig an einem Geräteaufstellfuß 3 angeordnet sind, wobei mindestens eine der Stapelhalteplatten 1, 2 begrenzt schwenkbar am Gerätefuß 3 gelagert ist und mit der anderen Stapelhalteplatte in Parallel- und Öffnungsstellung durch je ein an den Seitenrändern 4 der Stapelhalteplatten 1, 2 angeordnetes Verrastungselementenpaar 5 fixierbar ausgebildet ist, und daß ferner mindestens an einer der beiden Stapelhalteplatten 1, 2 innenseitig ein elastisch-flächenwirksames Druckelement 6 angeordnet ist.

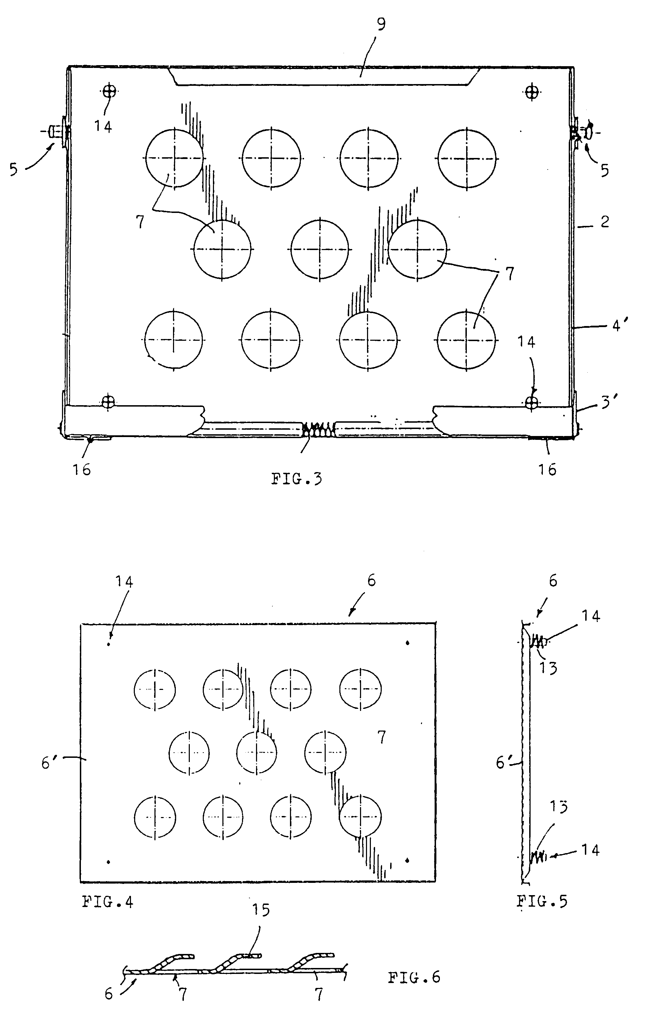

Bei der dargestellten und bevorzugten Ausführungsform ist die Stapelhalteplatte 1 senkrecht zum Gerätefuß 3 stehend fest mit diesem verbunden, und die andere Stapelhalteplatte 2 kann um das Gelenk 18 bis in die in Fig. 2 dargestellte Position aufgeschwenkt werden.

Unter Verweis auch auf Fig. 3 ist der Geräteaufstellfuß 3 aus zwei an den unteren Seitenrändern 4' der Stapelhalteplatten 1, 2 angeordneten Winkelstücken 3' gebildet, die beidseitig über die Parallelstellungsebenen E der Stapelhalteplatten 1, 2 ragen.

Die oberen freien Ränder 1', 2' der Stapelhalteplatten 1, 2 sind unter Ausbildung von Plattengriffen 9, wie dargestellt, abgekröpft und entsprechend mit ihrer Breite B der Überstandsbreite B₁ der Winkelstücke 3'. Wie ohne weiteres vorstellbar, kann dadurch das Gerät auch horizontal orientiert aufgestellt werden, wobei der eine oder andere Plattengriff 9 zum Aufstellfuß wird.

Die Verrastungselementenpaare 5 sind jeweils aus einer an einem Seitenrand 4 der einen Stapelhalteplatte 1 schwenkbar gelagerten Lasche 10 und aus einem Stellzapfen 11 am Seitenrand 4 der anderen Stapelhalteplatte 2 gebildet, wobei jeder Stellzapfen 11 in eine Schlitzführung 12 der Laschen 10 eingreift. In Rücksicht auf eine einfache Anbringbarkeit der Laschen 10 und der Stellzapfen 11 sind die Seitenränder 4' beider Stapelhalteplatten 1, 2 zum Stapelaufnahmeraum 8 hin rechtwinklig zu den Stapelplattenebenen E₁ abgekröpft ausgebildet.

Das Druckelement 6 (siehe Fig. 4, 5) ist aus einer mit Durchbrechungen 7 versehenen Druckplatte 6' gebildet und diese ist mit Federn 13 belastet und mit Zapfen 14 geführt, an der fest mit dem Gerätefuß 3 verbundenen Stapelhalteplatte 1 elastisch gelagert. Eine andere Möglichkeit (siehe Fig. 6) ist damit gegeben, daß die fest am Gerätefuß 3 angeordnete Stapelhalteplatte 1 und das Druckelement 6 zu einem Bauteil zusammengefaßt sind, wobei die Stapelhalteplatte 1 flächendeckend mit aus dem Plattenmaterial ausgekröpften, eine Vielzahl von kleinen Druckelementen 6 bildenden elastischen Zungen 15 versehen ist.

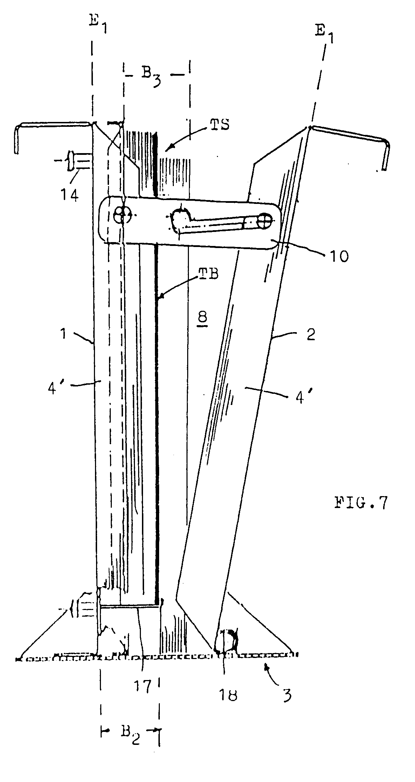

Eine besondere Ausführungsform ist in Fig. 7 verdeutlicht, gemäß der an der fest am Gerätefuß 3 angeordneten Stapelhalteplatte 1 in Distanz über dem bodenseitigen Teil 16 des Gerätefußes 3 eine Stapelauflage 17 angeordnet ist, die in ihrer Breite B₂ der halben Breite B₃ des Stapelaufnahmeraumes 8 entspricht.

Beim Einsetzen des Teststapels TS in den Stapelaufnahmeraum 8 ergibt sich dabei, wie dargestellt, ein Stufenversatz für die beiden Stapelhälften, wodurch, wie ebenfalls ersichtlich, das eigentliche Testblatt TB einem Zugriff direkt zugänglich wird. Eine solche Stapelauflage 17 kann aber auch, was nicht besonders dargestellt ist, ohne weiteres Teil des Gerätefußes 3 bzw. der Winkelstücke 3' sein.

## Patentansprüche

1. Gerät zum Testen von Dampfsterilisatoren mit Hilfe eines Testmaterialstapels, der im Inneren einen Testmaterialbogen enthält, bestehend aus zwei mit Durchbrechungen (7) versehenen, den Stapelaufnahmeraum (8) begrenzenden Stapelhalteplatten (1, 2), die relativ zueinander und in bezug auf den Testmaterialstapel in Parallel- und Spannstellung bring- und fixierbar sind,
**dadurch gekennzeichnet,**
daß die Stapelhalteplatten (1, 2) buchdeckelartig einander zugeordnet und buchdeckelrückseitig an einem Geräteaufstellfuß (3) angeordnet sind, wobei mindestens eine der Stapelhalteplatten (1, 2) begrenzt schwenkbar am Gerätefuß (3) gelagert ist und mit der anderen Stapelhalteplatte in Parallel- und Öffnungsstellung durch je ein an den Seitenrändern (4) der Stapelhalteplatten (1, 2) angeordnetes Verrastungselementenpaar (5) fixierbar ausgebildet ist, und daß ferner mindestens an einer der Stapelhalteplatten (1, 2) innenseitig ein elastisch-flächenwirksames Druckelement (6) angeordnet ist.

2. Gerät nach Anspruch 1,
**dadurch gekennzeichnet,**
daß der Geräteaufstellfuß (3) aus zwei an den unteren Seitenrändern (4') der Stapelhalteplatten (1, 2) angeordneten Winkelstücken (3') gebildet ist, die beidseitig über die Parallelstellungsebenen (E) der Stapelhalteplatten (1, 2) ragen.

3. Gerät nach Anspruch 1 oder 2,
**dadurch gekennzeichnet,**
daß die oberen freien Ränder (1', 2') der Stapelhalteplatten (1, 2) unter Ausbildung von Plattengriffen (9) abgekröpft sind und mit ihrer Breite (B) der Überstandsbreite (B₁) der Winkelstücke (3') entsprechen.

4. Gerät nach einem der Ansprüche 1 bis 3,
**dadurch gekennzeichnet,**
daß die Verrastungselementenpaare (5) jeweils aus einer an einem Seitenrand (4) der einen Stapelhalteplatte (1) schwenkbar gelagerten Lasche (10) und aus einem Stellzapfen (11) am Seitenrand (4) der anderen Stapelhalteplatte (2) gebildet sind, wobei der Stellzapfen (11) in eine Schlitzführung (12) der Lasche (10) eingreift.

5. Gerät nach einem der Ansprüche 1 bis 4,
**dadurch gekennzeichnet,**
daß das Druckelement (6) aus einer mit Durchbrechungen (7) versehenen Druckplatte (6') gebildet und diese, mit Federn (13) belastet und mit Zapfen (14) geführt, an der fest mit dem Gerätefuß (3) verbundenen Stapelhalteplatte (1) elastisch gelagert ist.

6. Gerät nach einem der Ansprüche 1 bis 4,
**dadurch gekennzeichnet,**
daß die fest am Gerätefuß (3) angeordnete Stapelhalteplatte (1) und das Druckelement (6) zu einem Bauteil zusammengefaßt sind, wobei die Stapelhalteplatte (1) flächendeckend mit aus dem Plattenmaterial ausgekröpften, eine Vielzahl von kleinen Druckelementen (6) bildenden elastischen Zungen (15) versehen ist.

7. Gerät nach einem der Ansprüche 1 bis 6,
**dadurch gekennzeichnet,**
daß an der fest am Gerätefuß (3) angeordneten Stapelhalteplatte (1) oder am Gerätefuß (3) in Distanz über dem bodenseitigen Teil (16) des Gerätefußes (3) eine Stapelauflage (17) angeordnet ist, die in ihrer Breite (B₂) der halben Breite (B₃) des Stapelaufnahmeraumes (8) entspricht.

8. Gerät nach einem der Ansprüche 1 bis 7,
**dadurch gekennzeichnet,**
daß die Seitenränder (4') beider Stapelhalteplatten (1, 2) zum Stapelaufnahmeraum (8) hin rechtwinklig zu den Stapelplattenebenen (E₁) abgekröpft sind.

## Claims

1. An assembly for testing steam sterilizers with the aid of a test material pile containing, in the interior thereof, a sheet of test material comprising two pile holding plates (1,2) provided with perforations (7) and confining the pile-accommodating space (8), with the pile holding plates (1,2) being able to be placed and liked in parallel and clamping positions relative to one another and in relation to the pile of test material,
characterized in that the pile holding plates (1,2) are associated to one another in the form of book covers and, on the book spine, are arranged on a mounting foot (3) of the assembly, with at least one of the pile holding plates (1,2) being arranged on the mounting foot (3) of the assembly in a manner swivable to a limited extent, and with the other pile holding plate being designed to be fixed in the parallel and opening positions through respectively one pair of locking elements (5) each arranged on the lateral edges (4) of the pile holding plates (1,2), and that, moreover, a resilient, surface-effective press element (6) is arranged on at least one of the pile holding plates (1,2).

2. An assembly according to claim 1, characterized in that the mounting foot (3) is formed of two elbow pieces (3') arranged on the bottom lateral edges (4') of the pile holding plates (1,2) which, on both sides, protrude beyond the parallel position planes (E) of the pile holding plates (1,2).

3. An assembly according to claims 1 or 2,
characterized in that the upper free edges (1', 2') of the pile holding plates (1,2) are cranked in forming plate handles (9), with the width (B) thereof corresponding to the projecting width (B₁) of the elbow pieces (3').

4. An assembly according to any one of claims 1 to 3,
characterized in that the pairs of locking elements (5) are respectively formed of a flap (10) swivably arranged on a lateral edge (4) of one of the pile holding plates (1), and of a mounting pin (11) arranged on the lateral edge (4) of the other of said pile holding plates (2), with the mounting pin (1) engaging a slit guide (2) of the flap (10).

5. An assembly according to any one of claims 1 through 4,
characterized in that the press element (6) is formed of a pressure plate (6') provided with perforations (7), and that the same are spring-loaded (13) and pin-guided (14) to be resiliently arranged on the pile holding plate (1) rigidly connected to the mounting foot (3) of the assembly.

6. An assembly according to any one of claims 1 through 4,
characterized in that the pile holding plate (1) rigidly arranged on the mounting foot (3) of the assembly and the press element (6) are combined to form a unit, with the pile holding plate (1), throughout the surface, are provided with flexible tongues cranked from the plate material and forming a multiplicity of small press elements (6).

7. An assembly according to any one of claims 1 through 6,
characterized in that a pile support (17) is arranged on the pile holding plate (1) rigidly arranged on the mounting foot (3) of the assembly or on the mounting foot (3) of the assembly at a distance from the bottom-sided part (16) of the mounting foot (3) of the assembly, with the width (B₂) of the pile support corresponding to half the width (B₃) of the pile-accommodating space (8).

8. An assembly according to any one of claims 1 through 7,
characterized in that the lateral edges (4') of both pile holding plates (1,2) toward the pile-accommodating space (8) are cranked at right angles relative to the pile plate planes (E₁).

## Revendications

1. Appareil pour tester des stérilisateurs à vapeur à l'aide d'une pile de matière de test, qui contient à l'intérieur une feuille de matière de test, constitué par deux plaques de maintien de pile (1, 2) pourvues de découpures (7), qui délimitent l'espace de logement de la pile (8), plaques qui peuvent être amenées et fixées en position parallèle et en position de serrage l'une par rapport à l'autre et par rapport à la pile de matière de test,
caractérisé en ce
que les deux plaques de maintien de pile (1, 2) se correspondent à la manière de la couverture d'un livre et qu'elles sont placées du côté du dos de la couverture du livre sur un pied pour mise en place debout de l'appareil (3), au moins l'une des plaques de maintien de la pile (1, 2) étant positionnée pivotante sur le pied de l'appareil (3) et étant configurée en pouvant être fixée avec l'autre plaque de maintien de la pile en position parallèle et en position d'ouverture par respectivement une paire d'éléments d'enclenchement (5) placés sur les bords latéraux (4) des plaques de maintien de la pile (1, 2), et que, de plus, un élément de pression (6) élastique, efficace en surface, est placé au moins sur l'une des deux plaques de maintien de la pile (1, 2) sur le côté intérieur.

2. Appareil selon la revendication 1,
caractérisé en ce
que le pied de mise en place debout de l'appareil (3) est formé par deux pièces coudées (3'), placées sur les bords latéraux inférieurs (4') des plaques de maintien de la pile (1, 2), qui font saillie des deux côtés au-delà des plans de position parallèle (E) des plaques de maintien de pile (1, 2).

3. Appareil selon la revendication 1 ou 2,
caractérisé en ce
que les bords libres supérieurs (1', 2') des plaques de maintien de pile (1, 2) sont coudés en formant des poignées de plaques (9) et correspondent, avec leur largeur (B), à la largeur de salle (B₁ ) des pièces coudées (3').

4. Appareil selon l'une des revendications 1 à 3,
caractérisé en ce
que les paires d'éléments d'enclenchement (5) sont formées respectivement par une attache (10) positionnée pivotante sur un bord latéral (4) de l'une des plaques de maintien de pile (1) et par un tourillon de réglage (11) sur le bord latéral (4) de l'autre plaque de maintien de pile (2), le tourillon de réglage (11) s'engrenant dans un guidage à rainure (12) de l'attache (10).

5. Appareil selon l'une des revendications 1 à 4,
caractérisé en ce
que l'élément de pression (6) est formé par une plaque de pression (6') pourvue de découpures (7) et celle-ci est chargée par des ressorts (13), guidée avec des tourillons (14) et positionnée élastique sur la plaque de maintien de pile (1) reliée de manière fixe au pied de l'appareil (3).

6. Appareil selon l'une des revendications 1 à 4,
caractérisé en ce
que la plaque de maintien de pile (1), placée de manière fixe sur le pied de l'appareil (3), et l'élément de pression (6) sont réunis en un composant, la plaque de maintien de pile (1) étant pourvue, de manière couvrant sa surface, de languettes élastiques (15) coudées dans la matière des plaques, qui forment une pluralité de petits éléments de pression (6).

7. Appareil selon l'une des revendications 1 à 6,
caractérisé en ce
qu'un support pour pile (17) est placé sur la plaque de maintien de pile (1), placée de manière fixe sur le pied de l'appareil (3), à une certaine distance au-dessus de la partie côté fond (16) du pied de l'appareil (3), support qui correspond, quant à sa largeur (B₂), à la moitié de la largeur (B₃) de l'espace de logement de la pile (8).

8. Appareil selon l'une des revendications 1 à 7,
caractérisé en ce
que les bords latéraux (4') des deux plaques de maintien de pile (1, 2) sont coudés vers l'espace de logement de la pile (8) à angles droits avec les plans des plaques de pile (E₁).
